# EUROPEAN PATENT APPLICATION

(11) **EP 4 239 643 A1**
(43) Date of publication of application: **06.09.2023**
(21) Application number: 23020105.5
(22) Date of filing: 03.03.2023
(51) Int. Cl.: G16H 20/60, G16H 10/20, G16H 10/60, G16H 20/30, G16H 20/70, G16H 50/30, G16H 10/40, G16H 15/00, G16H 40/67, G16H 50/20, G16H 50/70

(54) **AUTOMATED PERSONALIZED NUTRITION AND LIFESTYLE RECOMMENDATION SYSTEM AND ASSOCIATED METHOD THEREOF**

(30) Priority: 04.03.2022 US 202263316444 P
(71) Applicant: Mehdi, Mohsin, 1095 MX Amsterdam (NL)
(72) Inventor: Mehdi, Mohsin, 1095 MX Amsterdam (NL)

(57) **Abstract**

Disclosed is an automated personalized nutrition and lifestyle recommendations system and method. The system includes a data acquisition module for collecting genotype and phenotype data, historical diagnosis and medication data, and environmental parameters. The system includes a server configured to store personal information and information from the data acquisition module, and a processing unit for analyzing collected data, determining and storing required nutrition value at the molecular level, and determining and storing the best food that provides required nutrition value available in ROI, and determining and storing best lifestyle method for the end-user in the server. The system includes a user module that can access the data of end-users, a communication unit for establishing communication among the user module, the data acquisition module, and the bio-information server. The system includes a computer-readable method for providing distinct graphical user interfaces to user of the user module and to an admin.

## Description

### CROSS-REFERENCE TO RELATED APPLICATIONS

This patent application claims the benefit of priority of U.S. Provisional Application No. 63/316,444, entitled "AN AUTOMATED PERSONALIZED NUTRITION AND LIFESTYLE RECOMMENDATIONS SYSTEM BASED ON TOTAL GENOTYPE AND PHENOTYPE INFORMATION AND METHOD THEREOF," filed 04-MAR-2022, which is hereby incorporated herein by reference in its entirety.

### TECHNICAL FIELD OF INVENTION

The present invention generally relates to the field of methods and systems of optimizing diet, nutrition, and lifestyle to enhance the health and well-being of an individual. In particular, the present invention relates to an automated personalized nutrition and lifestyle recommendation system for an individual based on total genotype and phenotype information related to the individual and the associated method for recommendation thereof.

### BACKGROUND

A genotype is the collection of genetic material which are inherited from parents and present in the DNA (deoxyribonucleic acid) of every living being. The analysis of deoxyribonucleic acid (DNA) has provided a wealth of information related to a particular species, population, pool of organisms, and even specific individuals. Continuous discoveries lead to the ever-increasing knowledge of genetic data. A phenotype is the composite of an organism's observable characteristics or traits, such as its morphology, development, biochemical or physiological properties, behavior, and products of behavior. The genotype has a direct impact on the phenotype and/or phenotype can be abstracted based on the genotype and interaction of an individual with its surrounding.

There are numerous examples of genotype-phenotype relationships in humans in diverse areas including, e.g., medical/health, fitness/sport, aptitudes, nutrition, physical/physiological characteristics, psychological/mental, etc. phenotypes spanning the areas of medical/health, fitness/sport, aptitudes, nutrition, physical/physiological characteristics, psychological/mental, etc. These encompass susceptibility/risk (e.g., to injury or disease), rates (e.g., faster recovery or adaptiveness, metabolism), quantity (e.g., bone density, cardiac capacity), or binary (e.g., absence of wisdom teeth or presence of an enzyme), reaction (e.g., to drugs, alcohol, caffeine), etc.

A number of different types of systems and methods for diet and nutrition recommendation and enhancing lifestyle are available in the prior art. For example, US11244752B2 discloses an invention which is associated with systems and methods for recommending foods to a user based on health data, and includes a database, a memory, and a processor. The database stores user health data for each user within a plurality of users, including vitals, genotypic and phenotypic data, user food preference data, and foods data that includes macronutrient and micronutrient data for foods that may be recommended to a user. The memory stores program instructions, including program instructions that are capable of classifying user health data into predetermined diet types and micronutrient recommendations, filtering the food data to determine available foods for a user; and a ranking available meals for the user based on the micronutrient recommendations and the food data, and translating micronutrient recommendations and/or food data for the available foods for the user into specific food recommendations for the user.

Another prior art document, KR102221784B1 discloses an automation method for supplying customized menus extracted based on information of certain food materials related to gender, age, weight, height, information of high-risk metabolic disease groups based on metabolic disease status and genetics, and information of allergy status and certain food materials related to the allergy and based on the accessibility of the menu chosen by the user, type of the menu, etc. In this invention, the user can not only reduce a time spent for looking related publications with profession or cost for professional counseling but also can increase health promotion and improve effect of the disease.

Another prior art document, KR102346099B1 discloses a method of obtaining personal genetic information from the user's genetic test result, examines the user's body and body type, and allows to prescribe a personalized diet and exercise guide based on the obtained personal genetic information and body type test result. The invention includes: a genetic information setting unit configured to receive a user's genetic test result and set genetic information; a body information setting unit configured to receive the user's in-body test result and set body information; a control unit that analyzes the genetic information to determine and provide obesity risk index, three major nutrient metabolism, diet type, and recommended nutritional components, and prescribes an exercise guide based on the determined ones; an output device for outputting the determined obesity risk index, three major nutrient metabolism, diet type, recommended nutritional component, and the prescribed exercise guide on a screen and audio; a smart terminal in which a healthcare-related application is installed, the healthcare-related application is executed by the user, and NFC tagging is performed; and an NFC tag device that interworks with the output device and shares with the output device and the smart terminal by having an access address flag area in which an access address is recorded.

Another prior art document, CA2726958C discloses a system, method, and computer program for integrated, personalized disease management is provided. The method involves capturing individual attributes, analyzing the individual attributes to establish an individual classification for an individual, and based on the individual classification for the individual assigning automatically a program template for disease management of individual. The system provides a coaching platform for managing interactions between a coach and a system client based on the program template.

Another prior art document, WO2010017520A1 discloses methods and systems for personal action plans based on an individual's genomic profile. Methods include assessing the association between an individual's genotype and at least one disease or condition and providing rating systems for an individual's action plan. Incentives to motivate and encourage people to improve their health and well-being.

Another prior art document, AU2009246199B2 introduces a method and tests that allow for the establishment of personalized weight-loss programs for a subject based on the subject's metabolic genotype in key metabolic genes. Kits and methods are disclosed for determining a subject's metabolic genotype, which may be used to select an appropriate therapeutic/dietary regimen or lifestyle recommendation based on the likelihood of a subject's responsiveness to certain diets and activity levels. Such a personalized weight-loss program will have obvious benefits (e.g., yield better results in terms of weight loss and weight maintenance) over traditional weight-loss programs that do not take into account genetic information.

Another prior art document, US 10614724B2 discloses systems and methods for wellness, health, and lifestyle planning, tracking, and maintenance are provided. In general, the systems and methods described herein can allow a person to manage his/her wellness, health, and lifestyle using a convenient system that can help the person plan strategies for improving and/or maintaining his/her wellness, health, and lifestyle and/or that can help the person track his/her compliance with the strategies. In an exemplary embodiment, the system can be configured to provide recommendations of activities to the person that can positively affect the person's wellness, health, and lifestyle. The recommendations can be tailored to each individual user of the system such that different people can receive different recommendations.

Another prior art document, US8930204B1 discloses a computer-implemented method for determining lifestyle recommendations including receiving lifestyle information and healthcare information corresponding to an individual from a personal information aggregator. Lifestyle recommendations are determined based on the lifestyle information and the healthcare information and are provided to the individual.

Above mentioned references and many other similar references have one or more of the following shortcomings: (a) fail to provide a real-time analysis and recommendation; (b) doesn't provide nutrition value based on genotype and phenotype information; (c) fails to suggest about personalized nutrition and lifestyle plan; (d) less reliable; and (e) are not result oriented and inefficient.

The proposed invention addresses the above-mentioned concerns and shortcomings (and other similar concerns/shortcomings) through an automated personalized nutrition and lifestyle recommendation system and method, that operates based on total genotype and phenotype information associated with an individual.

### SUMMARY

In the view of the foregoing shortcomings inherent in the known systems and methods for providing nutrition and lifestyle recommendations, the inventor herein provides an improved system and method to enhanced nutrition and recommendation by providing accurate, efficient personalized nutrition and lifestyle plans which can be customized according to user necessity and condition. As such, the general purpose of the present invention, which will be described subsequently in greater detail, is to provide a new and improved nutrition and lifestyle recommendations system based on total genotype and phenotype information associated with a person/individual and a method thereof which can be used by any person, male or female regardless of age, environment geography.

According to one aspect of the present invention there is provided an automated personalized nutrition and lifestyle recommendations system based on total genotype and phenotype information associated with an individual/end user. The system comprises a data acquisition module for collecting genotype and phenotype data, historical diagnosis and medication data, and environmental parameters. The data acquisition module further comprises a genetic data acquisition unit, a physiological data acquisition unit, a medication and diagnoses data acquisition unit, an environmental parameter data acquisition unit, and a nutrition content data acquisition unit. The system further includes at least one decentralized bio-information server configured to store personal information and information from the data acquisition module in a block for each authorized end-user, and at least one processing unit for analyzing each value collected by each unit of the data acquisition module, determining and storing required nutrition value at the molecular level and determining and storing the best food that provides required nutrition value available in ROI, and determining and storing best lifestyle method for the end-user in bio-information server. The system further includes a user module that can access data of at least one or multiple end-users from the bio-information server, a communication unit for secured communication among the user module, data acquisition module, and decentralized bio-information server. The system also includes a computer-readable method to provide a distinct graphical user interface designed for each user of the user module and authorized admin.

According to the same aspect, the environmental parameters include but not limited to room temperature, humidity, pollution, food habit in the area, sociological factors, and phycological factors.

According to the same aspect, the user module includes at least one medical expert, a laboratory technician, a consultant, an insurance stakeholder, and other authorized people

According to the same aspect, the computer-readable method in the program product is stored in the form of executable instruction in the server, which when executed provides a distinct graphical user interface to each user with different content and is operable through the communication unit using any suitable operable devices (Eg. PCs, Phones).

According to another aspect of the present invention there is provided a method for automatic personalized nutrition and lifestyle recommendations based on total genotype and phenotype information. The method involves steps of registration and authorization, of each user of the user module and an authorized admin; collection, of at least genotype and phenotype data, historical diagnosis and medication data, environmental parameters, and nutrition available information related to the end-user by a data acquisition module; processing the collected genotype and phenotype data, historical diagnosis and medication data, environmental parameter, and nutrition information related to the end user by a processing unit; storing collected genotype and phenotype data, historical diagnosis and medication data, environmental parameter, and nutrition information related to the end user and processing the same in a bio-information server; identification of biomarkers and validation of biomarkers; storing the biomarkers with a threshold range in the bio-information server; comparison of each biomarker with data stored in bio-information server by the procession unit; generating one or more deficit reports when the value of data from the data acquisition module is out of the threshold range of the biomarkers, and displaying the current value of biomarkers of the end-user in the GUI of the end-user associated with a computer-readable method; calculating individual nutrition and lifestyle plans according to the one or more deficit reports considering historical diagnosis and medication data, environmental parameter, using an AI engine; displaying multiple personalized nutrition and lifestyle plans in the GUI of the end-user; selecting at least one of the personalized nutrition and lifestyle plans by the end-user; and delivering personalized nutrition and personalized supplements according to the nutrition plan selected by the end-user.

According to this aspect, the method of registration and authorization of every user of the user module begins with self-registration by entering preferred login credential in a GUI designated to each user of the user module and verification and authentication of login credential and user by an authorized admin. Next, on successful verification, and authentication, the user is authorized and the login credentials and personnel information is stored in the bio-information server.

According to the same aspect, the method of collecting genotype and phenotype includes obtaining, a genetic sample of the first end-user by a laboratory technician in any preferred location of the first end-user and testing the genetic sample to find every genetic information of the first end-user, the laboratory technician can store/enter genetic information to bio-information server through the GUI of the computer-readable medium in the first block. Entering phenotype information by the first end-user through end-user-specific GUI of computer-readable medium based on the questionary set by a processing unit and storing phenotype information of the first end-user in the first block.

According to the same aspect, in the method of historical diagnosis and medication data in which the first end-user enters details of historical diagnosis and media referencing medication details through end-user-specific GUI. The details of historical diagnosis are entered by diagnosing medical experts through medical expert-specific GUI. The details of historical diagnosis from the first end-user and diagnosing medical expert are validated by the processing unit and validated details of historical diagnosis is stored in the first block in the bio information server.

According to the same aspect, the method of nutrition available in the end-user of the preferred area comprises the following steps: a collection of details of available food items in the preferred location from any open-source server by the processing unit, fining molecular level nutrition value from details of available food items, mapping each molecular level nutrition value with a plurality of matrices corresponding to each food item, and storing each matrix in the bio-information server.

According to the same aspect, the method of calculating individual nutrition and lifestyle plans calculates multiple personalized nutrition and lifestyle plans based on the different time frames, e.g., for 1 month, for three months or can be customized according to user desired plan as end-user can choose a change in lifestyle for 2 hours daily, 5 hours daily.

Various advantages, parts, and features of the present invention will be described herein with specificity so as to make the present invention understandable to one of ordinary skill in the art, both with respect to how to practice the present invention and how to make the present invention.

### BRIEF DESCRIPTION OF THE DRAWINGS

So that the manner in which the above-recited features of the present invention can be understood in detail, a more particular description of the invention, briefly summarized above, may have been referred by embodiments, some of which are illustrated in the appended drawings. It is to be noted, however, that the appended drawings illustrate only typical embodiments of this invention and are therefore not to be considered limiting of its scope, for the invention may admit to other equally effective embodiments.

These and other features, benefits, and advantages of the present invention will become apparent by reference to the following text figure, with like reference numbers referring to like structures across the views, wherein:
FIG. 1 illustrates a block diagram of an automated personalized nutrition and lifestyle recommendations system based on total genotype and phenotype information, according to an embodiment of the present invention.
FIG. 2 depicts a flow chart of the method for automatic personalized nutrition and lifestyle recommendations based on total genotype and phenotype information, according to an embodiment of the present invention.
FIG. 3 depicts a flow chart of the method of registration and authorization of every user of the user module, according to an embodiment of the present invention.
FIG. 4 depicts a flow chart of the method of collecting genotype and phenotype, according to an embodiment of the present invention.
FIG. 5 depicts a flow chart of the method of historical diagnosis and medication data, according to an embodiment of the present invention.
FIG. 6 depicts a flow chart of the method of nutrition available in the end-user preferred area, according to an embodiment of the present invention.

### DETAILED DESCRIPTION OF THE INVENTION

In the following detailed description, reference is made to the accompanying drawings which form a part hereof, and in which is shown by way of illustration specific embodiment in which the invention may be practiced. These embodiments are described in sufficient detail to enable those skilled in the art to practice the invention, and it is to be understood that the embodiments may be combined, or that other embodiments may be utilized and that structural and logical changes may be made without departing from the spirit and scope of the present invention. The following detailed description is, therefore, not to be taken in a limiting sense, and the scope of the present invention is defined by the appended claims and their equivalents.

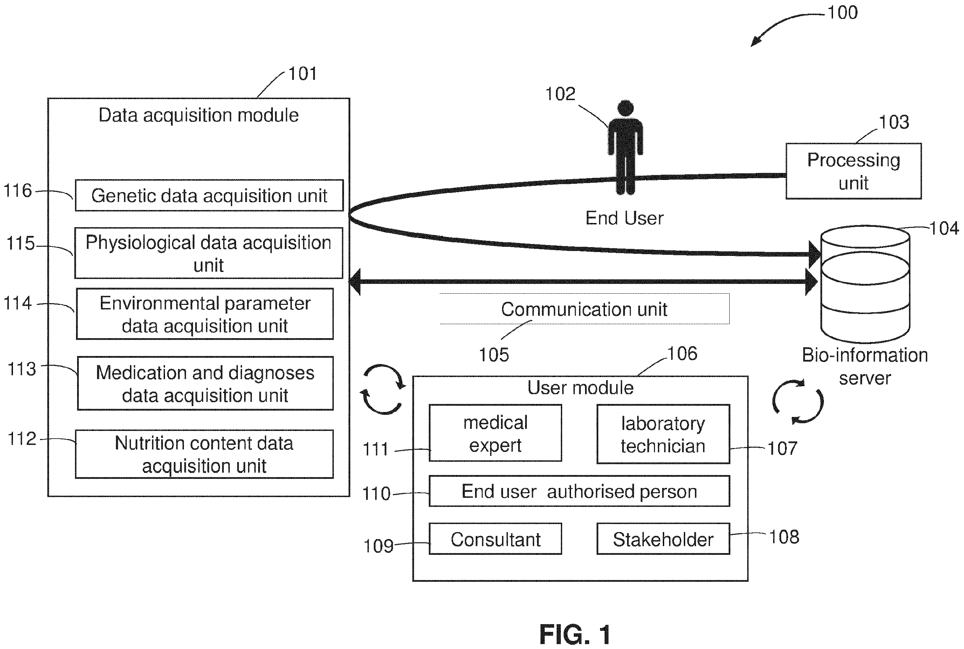

References will now be made in detail to the exemplary embodiment of the present disclosure. Before describing the detailed embodiments that are in accordance with the present disclosure, it should be observed that the embodiments reside primarily in combinations arrangement of the system according to an embodiment herein and as exemplified in **FIGS. 1-6****.**

In the following description, for the purpose of explanation, numerous specific details are set forth in order to provide a thorough understanding of the arrangement of the system according to an embodiment herein. It will be apparent, however, to one skilled in the art, that the present embodiment can be practiced without these specific details. In other instances, structures are shown in block diagram form only in order to avoid obscuring the present invention.

The main embodiment of the present invention is to provide an automated personalized nutrition and lifestyle recommendations system based on total genotype and phenotype information and the method thereof. FIG. 1 shows a block diagram of an automated personalized nutrition and lifestyle recommendations system 100 (referred to as "system 100" hereinafter) based on total genotype and phenotype information. The system 100 based on total genotype and phenotype information includes a data acquisition module 101 for collecting genotype and phenotype data, historical diagnosis and medication data, and environmental parameters. The data acquisition module 101 further includes a genetic data acquisition unit 116, a physiological data acquisition unit 115, a medication and diagnoses data acquisition unit 113, an environmental parameter data acquisition unit 114, and a nutrition content data acquisition unit 112. The system 100 further includes at least one decentralized bio-information server 104 configured to store personnel information and information from the data acquisition module 101 in a block for each authorized end-user. The server 104 may be any general-purpose server with computing capabilities such as pc, or computer. The server 104 may be a cloud-based server. The system further includes at least one processing unit 103 configured for analyzing each value collected by each unit of the data acquisition module 101, determining and storing required nutrition value at the molecular level, and determining and storing the best food that provides required nutrition value available in ROI and determining and storing best lifestyle method for the end-user 102 in the bio-information server 104. According to the embodiment, the system 100 includes a user module 106 that can access data of at least one or multiple end-users from the bio-information server 104. The system 100 further shows a communication unit 105 that helps in establishing a secure communication among the user module 106, the data acquisition module 101, and the decentralized bio-information server. The system further includes a computer-readable method or computer readable media/software product that provides a distinct graphical user interface to each user of the user module and authorized admin.

According to the embodiment, the environmental parameter may include but not limited to room temperature, humidity, pollution, food habit in the area, sociological factors, and phycological factors. The environmental factor impacts the phenotype information and also the healthy lifestyle, for example, doing exercise in low-pollution areas at any time of the day is far better than doing physical exercises in the early morning in highly polluted areas. The health and other wellness of a person/individual/end user also depends upon the food habit of a region or area.

The user module 106 in the context of the present invention includes at least one medical expert 111, at least one laboratory technician 107, a consultant 109, an insurance stakeholder 108, and other authorized people 110 by the end-user 102. The end-user herein is the person or user who is inclined to receive the nutrition recommendation and lifestyle recommendation, automatically using the automated personalized nutrition and lifestyle recommendations system 100 of the present invention. A consultant may be any person or authority that may provide medical consultation for any diseases that the end-users may be suffering from. The insurance stakeholder is the authority in which the end-user 102 may open or already have an insurance policy, in case of any requirement either the insurance stakeholder or the end-user directly provides or receives insurances service respectively.

A computer-readable method or computer -readable program product herein is stored set of executable instructions with a distinct graphical user interface designed for each user with different content and operable through the communication unit 105 via user devices (not shown, but may include phones, tabs, etc.).

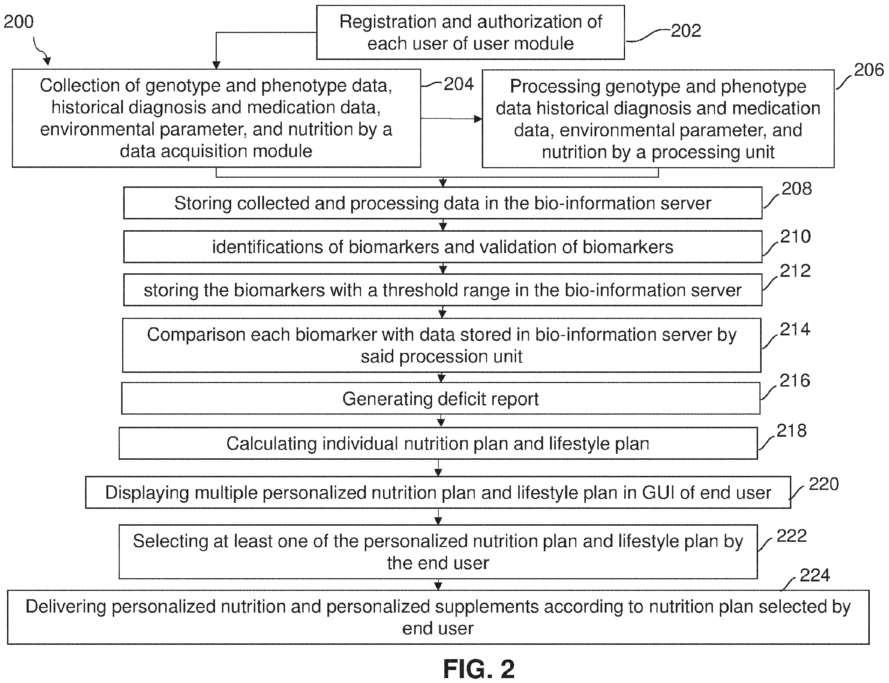

FIG. 2 shows a flow chart 200 of the method for automatic personalized nutrition and lifestyle recommendations based on total genotype and phenotype information, according to an embodiment herein. The method for automatic personalized nutrition and lifestyle recommendations based on total genotype and phenotype information initiate with registration and authorization (step 202), of each user of the user module 105 and the admin and on successful registration and authorization, a data acquisition module 101 collects genotype and phenotype data, historical diagnosis and medication data, environmental parameter, and nutrition available in the end-user preferred area or location (step 204). Collected data such as genotype and phenotype data, historical diagnosis and medication data, environmental parameters, and nutrition available in end-user 102 preferred area are processed by a processing unit 103 (step 206). Collected and processing data are stored in the bio-information server (step 208) and identifications of biomarkers and validation of biomarkers (step 210). The biomarkers with a threshold range are stored in the bio-information server (step 212) and comparison each biomarker with data stored in bio-information server 104 by the procession unit 103 (step 214) and followed by generating a deficit report (step 216) when the value of data from the acquisition module is out of the threshold range of the biomarkers and displaying the current value of biomarkers of the end-user in GUI of the end-user. The individual nutrition and lifestyle plans are calculated (step 218) according to the deficit report considering historical diagnosis and medication data, environmental parameters, using AI engine and displaying the multiple personalized nutrition plan and lifestyle plans in GUI of end-user (step 220). The end-user 102 selects at least one of the personalized nutrition plans and lifestyle plans (step 222) by the end-user and authorized admin/user module 106 delivers personalized nutrition and personalized supplements according to the nutrition plan selected by the end-user (step 224).

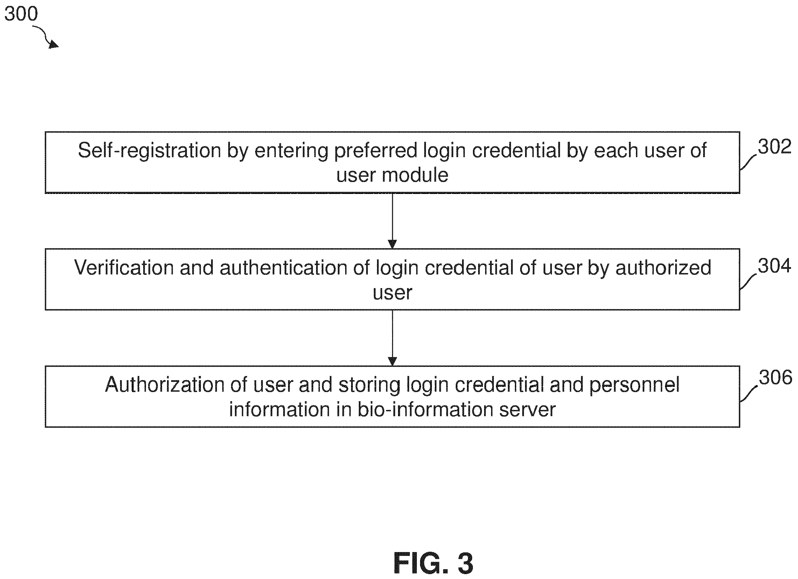

FIG. 3 shows a flow chart 300 of the method of registration and authorization of every user of the user module, according to an embodiment of the present invention. The method of registration and authorization of every user of user module 106 begins with self-registration by entering preferred login credentials in GUI designated to each user of the user module (step 302) and verification and authentication of login credentials and users by an authorized admin (step 304). And on successful verification and authentication user are authorized and store login credentials and personnel information in the bio-information server (step 306).

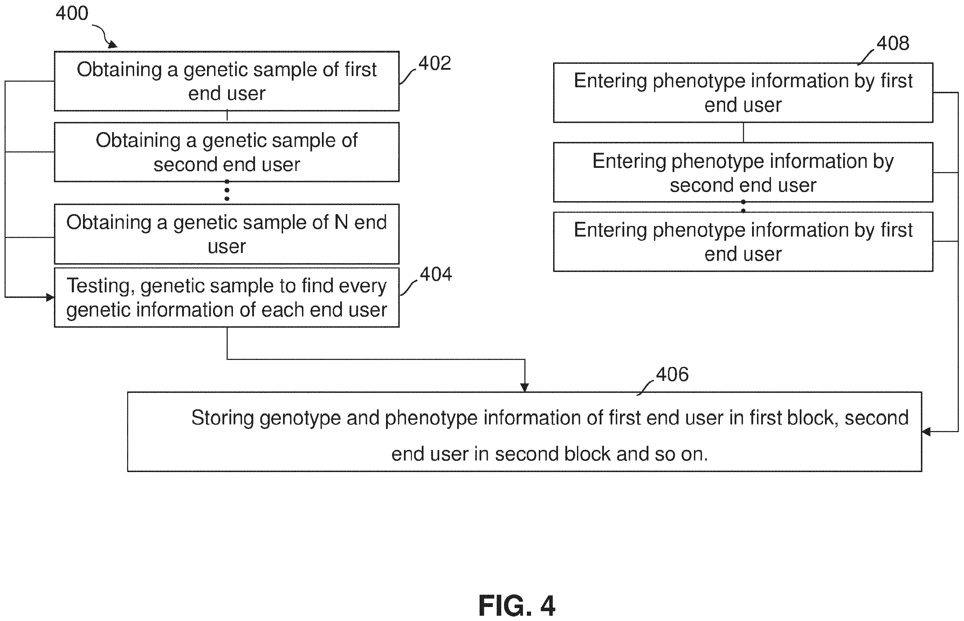

FIG. 4 shows a flow chart 400 of the method of collecting genotype and phenotype, according to an embodiment of the present invention. The method of collecting genotype and phenotype starts with obtaining, a genetic sample of the first end-user (step 402) by a laboratory technician 107 in any preferred location of the first end-user and testing the genetic sample to find every genetic information of the first end-user (step 404), laboratory technician 107 can store/enter genetic information to bio-information server 104 through the GUI of the computer-readable medium in the first block (step 406). Entering phenotype information by first end-user (step 408) through end-user 102 specific GUI of computer-readable medium based on the questionary set by a processing unit 103 and storing phenotype information of first end-user in the first block.

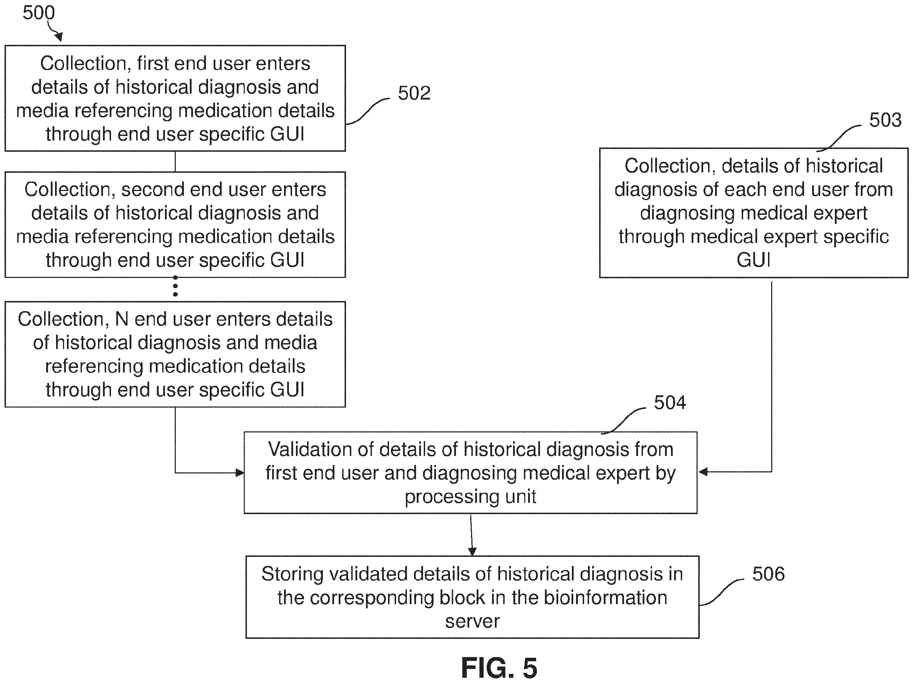

FIG. 5 shows a flow chart 500 of the method of historical diagnosis and medication data, according to an embodiment of the present invention. In the method of historical diagnosis and medication data in which the first end-user enters details of historical diagnosis and media referencing medication details through end-user-specific GUI (step 502). The details of historical diagnosis are entered by diagnosing medical expert 111 through medical expert specific GUI (step 503). The details of historical diagnosis from the first end-user and diagnosing medical expert are validated by processing unit 103 (step 504) and storing validated details of historical diagnosis in the first block in the bio information server (step 506).

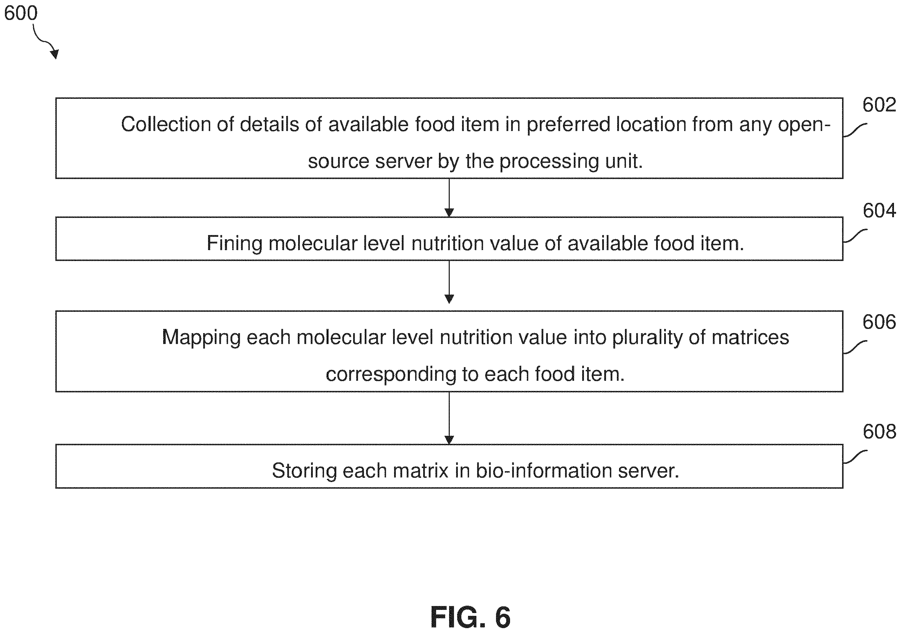

FIG. 6 shows a flow chart 600 of the method of nutrition available in the end-user preferred area, according to an embodiment of the present invention. The method of nutrition available in the end-user preferred area starts with collecting details of available food item in the preferred location from any open-source server by the processing unit 103 (step 602) and fining molecular level nutrition value from details of the available food item (step 604). Each molecular level nutrition value is mapped into a plurality of matrices corresponding to each food item (step 606) and stored in each matrix in the bio-information server (step 608).

The method of calculating individual nutrition plans and lifestyle plans calculates multiple personalized nutrition and lifestyle plans based on different time frames, e.g., for 1 month, for three months, or can be customized according to the user's desired plan as the end-user can choose a change in lifestyle for 2 hours daily, 5 hours daily.

It is to be understood that the above description is intended to be illustrative, and not restrictive. For example, the above-discussed embodiments may be used in combination with each other. Many other embodiments will be apparent to those of skill in the art upon reviewing the above description.

The benefits and advantages which may be provided by the present invention have been described above with regard to specific embodiments. These benefits and advantages, and any elements or limitations that may cause them to occur or to become more pronounced are not to be construed as critical, required, or essential features of any or all of the embodiments.

While the present invention has been described with reference to particular embodiments, it should be understood that the embodiments are illustrative and that the scope of the invention is not limited to these embodiments. Many variations, modifications, additions and improvements to the embodiments described above are possible. It is contemplated that these variations, modifications, additions and improvements fall within the scope of the invention.

## Claims

1. An automated personalized nutrition and lifestyle recommendations system (100) based on total genotype and phenotype information of an end user (102), comprising:
a data acquisition module (101) for collecting at least genotype and phenotype data, historical diagnosis and medication data, and environmental parameters, wherein the data acquisition module (101) further comprises a genetic data acquisition unit (116), a physiological data acquisition unit (115), a medication and diagnoses data acquisition unit (113), an environmental parameter data acquisition unit (114), and a nutrition content data acquisition unit (112);
at least one decentralized bio-information server (104) configured to store personal information associated with the end-user (102) and information from the data acquisition module (101);
at least one processing unit (103), wherein the processing unit (103) is configured for analyzing at least genotype and phenotype data, historical diagnosis and medication data, and environmental parameters collected by the data acquisition module (101), determining and storing required nutrition value at the molecular level, and determining and storing best food that would provide required nutrition value available in ROI, and determining and storing best lifestyle method for the end-user in the bio-information server (104);
a user module (106) capable of accessing personal information of the end-user from the bio-information server (104);
a communication unit (105) for a secured communication among the user module (106), the data acquisition module (101), and the bio-information server (104); and
a computer-readable method capable of presenting a distinct graphical user interface (GUI) to each user of the user module (106) and an authorized admin.

2. The automated personalized nutrition and lifestyle recommendations system (100) as claimed in claim 1, wherein the environmental parameters comprises at least room temperature, humidity, pollution, food habit in an area, sociological factors, and phycological factors.

3. The automated personalized nutrition and lifestyle recommendations system (100) as claimed in claim 1, wherein the user module (106) comprises at least one medical expert (111), at least one laboratory technician (107), at least one preferred consultant(109), at least one insurance stakeholder (108), and end-user authorized person (110).

4. The automated personalized nutrition and lifestyle recommendations system (100) as claimed in claim 1, wherein the computer-readable method comprises stored executable instruction with a distinct graphical user interface designed for each user with different content and operable through the communication unit (105).

5. A method for automatic personalized nutrition and lifestyle recommendations based on total genotype and phenotype information of an end user (102), comprising:
registration and authorization, of each user of the user module (106) and an authorized admin;
collection, of at least genotype and phenotype data, historical diagnosis and medication data, environmental parameters, and nutrition available information related to the end-user (102) by a data acquisition module (101);
processing the collected genotype and phenotype data, historical diagnosis and medication data, environmental parameter, and nutrition information related to the end user (102)by a processing unit (103);
storing collected genotype and phenotype data, historical diagnosis and medication data, environmental parameter, and nutrition information related to the end user (102) and processing the same in a bio-information server (104);
identification of biomarkers and validation of biomarkers;
storing the biomarkers with a threshold range in the bio-information server (104);
comparison of each biomarker with data stored in bio-information server (104) by the procession unit (103);
generating one or more deficit reports when the value of data from the data acquisition module (101) is out of the threshold range of the biomarkers, and displaying the current value of biomarkers of the end-user in the GUI of the end-user (102) associated with a computer-readable method;
calculating individual nutrition and lifestyle plans according to one or more deficit reports considering historical diagnosis and medication data, environmental parameters, using an AI engine;
displaying multiple personalized nutrition and lifestyle plans in the GUI of the end-user 102;
selecting at least one of the personalized nutrition and lifestyle plans by the end user (102); and
delivering personalized nutrition and personalized supplements according to the nutrition plan selected by the end-user (102).

6. The method for automatic personalized nutrition and lifestyle recommendations based on total genotype and phenotype information as claimed in claim 5, wherein the method of registration and authorization of every user of the user module (105) comprising the following steps:
self-registration by entering the preferred login credential in the GUI designated to each user of the user module (105);
verification and authentication of the login credentials of the user by the authorized admin; and
authorization of user on successful verification and authentication and storing login credentials and personnel information in the bio-information server (104).

7. The method for automatic personalized nutrition and lifestyle recommendations based on total genotype and phenotype information as claimed in claim 5, wherein the method of collecting genotype and phenotype initiated with
obtaining, a genetic sample of a first end-user (102) by a laboratory technician (107) in any preferred location of the first end-user (102);
testing, genetic sample to find every genetic information of first end-user, the laboratory technician capable of storing/entering genetic information into the bio-information server (104) through the GUI associated with the computer-readable medium ;
entering phenotype information by the first end-user through end-user-specific GUI of the computer-readable medium based on a questionnaire set by the processing unit (103); and
storing phenotype information of the first end-user in the first block.

8. The method for automatic personalized nutrition and lifestyle recommendations based on total genotype and phenotype information as claimed in claim 5, wherein the method of historical diagnosis and medication data comprising the following steps:
collection, of the first end-user enters details of historical diagnosis and media referencing medication details through end-user specific GUI;
collection, of details of historical diagnosis from diagnosing medical experts through medical expert-specific GUI;
validation of details of historical diagnosis from the first end-user and diagnosing medical expert by the processing unit (103); and
storing validated details of the historical diagnosis in the first block of the bio-information server (104).

9. The method for automatic personalized nutrition and lifestyle recommendations based on total genotype and phenotype information as claimed in claim 5, wherein said method of nutrition available in the end-user (102) of the preferred area comprising the following steps:
collection of details of available food items in the preferred location from any open-source server by the processing unit (103);
fining molecular level nutrition value from details of available food items;
mapping each molecular level nutrition value with a plurality of matrices corresponding to each food item; and
storing each matrix in the bio-information server (104).

10. The method for automatic personalized nutrition and lifestyle recommendations based on total genotype and phenotype information as claimed in claim 5, wherein the method of calculating individual nutrition and lifestyle plans calculates multiple personalized nutrition and lifestyle plans based on the different time frames.
